(19) Europäisches Patentamt
European Patent Office
Office européen des brevets

(11) **EP 3 776 575 B1**

(12) **EUROPEAN PATENT SPECIFICATION**

(45) Date of publication and mention
of the grant of the patent:
**08.06.2022 Bulletin 2022/23**

(21) Application number: **19714671.5**

(22) Date of filing: **03.04.2019**

(51) International Patent Classification (IPC):
**G16H 40/63** *(2018.01)* **G16H 20/17** *(2018.01)*

(52) Cooperative Patent Classification (CPC):
**G16H 20/17; G16H 40/63**

(86) International application number:
**PCT/EP2019/058416**

(87) International publication number:
**WO 2019/193061 (10.10.2019 Gazette 2019/41)**

(54) **DATA TRANSMISSION SAFETY FEATURE**

DATENÜBERTRAGUNGSSICHERHEITSMERKMAL

CARACTÉRISTIQUE DE SÉCURITÉ DE TRANSMISSION DE DONNÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2018 EP 18165422**

(43) Date of publication of application:
**17.02.2021 Bulletin 2021/07**

(73) Proprietor: **Novo Nordisk A/S
2880 Bagsværd (DK)**

(72) Inventor: **HOLM, Per, Einar, Pontus
2880 Bagsværd (DK)**

(74) Representative: **Kristoffersen, Bo
Novo Nordisk A/S
Corporate Patents
Novo Allé
2880 Bagsvaerd (DK)**

(56) References cited:
**WO-A1-2015/185687      US-A1- 2015 117 645
US-A1- 2017 201 931**

- **MAYRHOFER R ET AL: "Shake Well Before Use: Intuitive and Secure Pairing of Mobile Devices", IEEE TRANSACTIONS ON MOBILE COMPUTING, IEEE SERVICE CENTER, LOS ALAMITOS, CA, US, vol. 8, no. 6, 1 June 2009 (2009-06-01), pages 792-806, XP011335360, ISSN: 1536-1233, DOI: 10.1109/TMC.2009.51**

## Description

The present invention generally relates to methods and devices for wireless transmission of data from a data generating device, e.g. from medical devices for which the generation, collecting and storing of data are relevant. In a specific aspect the invention relates to methods providing secure and effective pairing of devices. In specific embodiments the invention relates to devices and systems for transmitting drug delivery dose data in a secure, reliable and user-friendly way.

## BACKGROUND OF THE INVENTION

In the disclosure of the present invention reference is mostly made to drug delivery devices comprising a threaded piston rod driven by a rotating drive member, such devices being used e.g. in the treatment of diabetes by delivery of insulin, however, this is only an exemplary use of the present invention. Alternatively the concepts of the present invention could be implemented in other drug delivery devices such as inhalers or nebulizers, or in data generating devices such as blood glucose meters (BGMs) or continuous blood glucose meters (CGMs).

Drug delivery devices have greatly improved the lives of patients who must self-administer drugs and biological agents. Drug Injection devices may take many forms, including simple disposable devices that are little more than an ampoule with an injection means or they may be durable devices adapted to be used with prefilled cartridges. Regardless of their form and type, they have proven to be great aids in assisting patients to self-administer injectable drugs and biological agents. They also greatly assist care givers in administering injectable medicines to those incapable of performing self-injections.

Performing the necessary insulin injection at the right time and in the right size is essential for managing diabetes, i.e. compliance with the specified insulin regimen is important. In order to make it possible for medical personnel to determine the effectiveness of a prescribed dosage pattern, diabetes patients are encouraged to keep a log of the size and time of each injection. However, such logs are normally kept in handwritten notebooks, and the logged information may not be easily uploaded to a computer for data processing. Furthermore, as only events, which are noted by the patient, are logged, the note book system requires that the patient remembers to log each injection, if the logged information is to have any value in the treatment of the patient's disease. A missing or erroneous record in the log results in a misleading picture of the injection history and thus a misleading basis for the medical personnel's decision making with respect to future medication. Accordingly, it may be desirable to automate the logging of injection information from medication delivery systems.

Correspondingly, some proposed drug delivery devices integrate this monitoring/acquisition mechanism into the device itself, e.g. as disclosed in US 2009/0318865, WO 2010/052275 and WO 2016/110592, these devices being of the durable type, whereas WO 2015/071354 discloses a disposable drug delivery device provided with dose logging circuitry.

However, most devices of today are without it. Addressing this problem a number of solutions have been proposed which would help a user to generate, collect and distribute data indicative of the use of a given medical device. For example, WO 2013/120776 describes an electronic supplementary device (or add-on device) adapted to be releasably attached to a drug delivery device of the pen type. The device includes a camera and is configured to perform optical character recognition (OCR) on captured images from a rotating scale drum visible through a dosage window on the drug delivery device, thereby to determine a dose of medicament that has been dialled into the drug delivery device. A further external device for a pen device is shown in WO 2014/161952, the external device being designed to determine dose sizes based on detection of movement of a magnetic member incorporated in the pen device.

Although the above-described logging devices in general are provided with a display allowing logged dose data to be displayed, it may be desirable to transfer dose data to an external device, e.g. a smartphone as carried by many drug delivery device users today, this allowing the dose data to be displayed on a much larger display and to be further processed and used for e.g. analysis and recommendations. Such an arrangement would also allow the display on the logging device to be dispensed with. A concept of wireless pairing a medical device with a smartphone is described in US 2017/0201931, the method using a tap event to pair. In document US 2015/117645 A1 the receiver and the transmitter generate the encryption keys entirely independent from each other and thereby does not disclose the features of the present invention in which the transmitter "hints" the receiver in the generation of the encryption key.

Having regard to the above, it is an object of the present invention to provide devices and methods allowing secure, easy and cost-effective wireless transfer of a data log from a data generating device, e.g. from a drug delivery device with dose logging capabilities to an external device such as a smartphone or smartwatch. It is a specific object of the invention to provide methods providing secure and effective pairing of devices. The present invention is defined by the appended claims.

## DISCLOSURE OF THE INVENTION

In the disclosure of the present invention, embodiments and aspects will be described which will address one or more of the above objects or which will address objects apparent from the below disclosure as well as from the description of exemplary embodiments.

Thus, in a general aspect of the invention a method is provided for wireless pairing of two devices for the subsequent secure transmission of data. Each device comprises impact sensor circuitry for detecting an impact event made to the device, and is adapted to determine a unique transmitter time related value based on the detected impact event, respectively a corresponding receiver time related value based on the detected impact event. The transmitting device is adapted to calculate and transmit a hint H for a key K based on the unique time related value, and the receiving device is adapted to calculate the key K based on the received hint H and the corresponding time related value. Alternatively, more than one time related value may be determined and used in the pairing process. As the pairing method in its basic form is based upon one-way transmission of data, strictly speaking, it could be said that only the receiving device via the key K is paired to the transmitting device, whereas the transmitting device will not be paired to any specific receiving device. Indeed, in specific embodiments two-way communication may be added for other purposes.

In a specific aspect of the invention a method of wireless pairing of two devices for subsequent secure transmission of data is provided. The method comprises the steps of providing a transmitter device adapted to wirelessly transmit data and a receiver device adapted to wirelessly receive data transmitted from the transmitter device. The transmitter device comprises transmitter impact sensor circuitry for detecting an impact event made to the transmitter device, and a transmitter clock, wherein, when an impact event having a pre-defined characteristic is detected by the transmitter impact sensor circuitry, the transmitter device is adapted to determine at least one unique time related value based on the detected impact event, and initiate a wireless pairing process. The receiver device comprises receiver impact sensor circuitry for detecting an impact event made to the receiver device, and a receiver clock, wherein, when an impact event having a pre-defined characteristic is detected by the receiver impact sensor circuitry, the receiver device is adapted to determine at least one corresponding time related value based on the detected impact event, and initiate a wireless pairing process. The transmitter device pairing process comprises the steps of retrieving or determining a key K for coding and decoding data, based on the key K and at least one unique time related value, calculating a hint H, and transmitting a data package comprising the hint H and including at least one unique time related value. The receiver device pairing process comprises the steps of receiving the data package transmitted from the transmitter device, and based on the hint H and at least one corresponding time related value, calculating and storing the key K. The method comprises the further step of impacting the first and second device against each other.

The term "corresponding" indicates that by the impact event a known or predetermined relationship is created between a determined unique time related value and a determined corresponding time related value. The pair of unique and corresponding time related values may be in the form of e.g. time stamps or other characteristics of the impact event as described in greater detail below.

By this arrangement a method is provided which allows a "secret" key to be transmitted wirelessly from a first device to a second device in a secure and user friendly way, e.g. an encryption key. As successful transfer of the key is based on a specific physical impact detected simultaneously by the impacted two devices the risk of an "outside" device being able to also determine the key is considered to be small.

In an exemplary embodiment of the method a determined unique time related value is a unique time stamp T1 and a determined corresponding time related value is a corresponding time stamp T2. The transmitter device pairing process comprises the further steps of starting a transmitter timer, determining a time delay value D, and after a time delay of D from start of the transmitter timer, transmitting the data package comprising the value (T1+D) and hint H. The receiver device pairing process comprises the further steps of starting a receiver timer, determining the time T when the data package is received, estimating D based on the difference between the time T and T2, calculating T1 as the difference between (T1+D) and the estimated D, and based on the hint H and the value T1, calculating and storing the key K.

In a further exemplary embodiment of the method the impact event comprises at least two impacts, e.g. a double tap, on the basis of which a unique time related value V1 and a corresponding time related value V2 are determined. The hint H is calculated by the transmitter device based on the unique time related value V1, and the key K is calculated by the receiver device based on the hint H and the corresponding time related value V2.

In a yet further exemplary embodiment of the method a unique time related value V1 and a corresponding time related value V2 are determined based on the waveforms of the detected impacts. The hint H is calculated based on the unique time related value V1, and the key K is calculated by the receiver device based on the hint H and the corresponding time related value V2.

In the above described exemplary embodiments a single pair of time related values are used, however, more than one pair of time related values may be determined based on a given impact event, this providing an even higher safety for the transmission of a given key. For example, a pair of time stamps may be combined with time related values based on other impact characteristics.

Correspondingly, the above-described examples using a double tap or the shapes of impact waveforms may be supplemented by a unique time stamp T1 and a corresponding time stamp T2 determined based on the detected impact events. The transmitter device pairing process may comprise the further steps of starting a transmitter timer, determining a time delay value D, calculating the hint H based on D and the unique time related value V1, and after a time delay of D from start of the transmitter timer, transmitting the data package comprising the value (T1+D) and hint H. The receiver device pairing process may comprise the further steps of starting a receiver timer, determining the time T when the data package is received, estimating D based on the difference between the time T and T2, calculating T1 as the difference between (T1+D) and the estimated D, and based on the hint H, the corresponding time related value V2 and the value T1, calculating and storing the key K.

The transmitter device pairing process may comprise the transmission of data encrypted with the key K. When a key K has been retrieved or determined by the transmitter device it may be stored and used for all subsequent transmission of encrypted data from that device. The transmitter device may initiate a wireless pairing process each time an impact having a pre-defined characteristic is detected by the transmitter impact sensor circuitry, whereas the receiver device may initiate a wireless pairing process only when set in a pairing state. Communication may take place using e.g. Bluetooth® or Bluetooth® Low Energy (BLE).

In a further aspect of the invention a transmitter device comprising electronic circuitry is provided. The electronic circuitry comprises wireless transmission means for wireless transfer of data to an external device, impact sensor circuitry for detecting an impact made to the transmitter device, and a clock. When an impact to the transmitter having a pre-defined characteristic is detected by the impact sensor circuitry, the transmitter device is adapted to determine at least one time related value based on the detected impact event, and initiate a wireless pairing process comprising the steps of retrieving or determining a key K for coding and decoding data, based on the key K and at least one time related value, calculating a hint H, transmitting a data package comprising the hint H and including at least one time related value. In exemplary embodiments the transmitter device may be specifically adapted corresponding to the above described methods.

The transmitter device may be in the form of a drug delivery device or a drug delivery assembly, further comprising a drug reservoir or means for receiving a drug reservoir, and drug expelling means comprising dose setting means allowing a user to set a dose amount of drug to be expelled, the electronic circuitry further comprising sensor means adapted to capture a property value related to the dose amount of drug expelled from a reservoir by the expelling means during an expelling event, and storage means adapted to store a plurality of property values to create a log. Alternatively, the transmitter device may be in the form of other drug delivery devices such as inhalers or nebulizers, or in data generating devices such as blood glucose meters (BGMs) or continuous blood glucose meters (CGMs).

In a yet further aspect of the invention a receiver device is provided, comprising electronic circuitry with wireless data receiving means, impact sensor circuitry for detecting an impact made to the receiver device, a clock, and a stored algorithm for calculating a key K based on a received hint H and an impact related time value. When an impact to the receiver device having a pre-defined characteristic is detected by the impact sensor circuitry, the receiver device is adapted to determine at least one time related value based on the detected impact event, and initiate a wireless pairing process comprising the steps of receiving a data package comprising a hint H, and based on a received hint H and at least one determined time related value, calculating and storing the key K. In exemplary embodiments the receiver device may be specifically adapted corresponding to the above described methods.

The receiver device may be in the form of a storage device further comprising storage means adapted to store received data (i.e. for a longer period), e.g. a wearable device such as a smartphone or a smartwatch, or a computer, e.g. a tablet computer, a laptop computer or a stationary computer. The computer may comprise or be associated with a separate receiver unit adapted to transmit data to the computer *per se*, e.g. wired or wirelessly. The storage device may be adapted to transmit data to a further device or to a cloud service.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin, however, the described module could also be used to create logs for other types of drug, e.g. growth hormone or drugs for haemophilia treatment.

## BRIEF DESCRIPTION OF THE DRAWINGS

In the following embodiments of the invention will be described with reference to the drawings, wherein

fig. 1 shows a first example in which an encryption key is being transmitted,
fig. 2 shows a second example in which an encryption key is being transmitted,
fig. 3 shows a third example in which an encryption key is being transmitted,
fig. 4A shows a first drug delivery device,
fig. 4B shows a flexible sheet with electronic circuitry,
fig. 5 shows a second drug delivery device, and
fig. 6 shows an add-on device mounted on a third drug delivery device.

In the figures like structures are mainly identified by like reference numerals.

## DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

The present invention addresses the general issue of securely, safely and user friendly associating (pairing) a data generating device with an external device for wireless communication there between.

In an exemplary embodiment safe and user friendly pairing of a data generating device with an external device is accomplished using a custom Bluetooth® Low Energy (BLE) radio chip. By removing the receiver part of the radio the size and complexity of the radio chip can be significantly reduced and thus the cost. Such a radio chip may be incorporated in a drug delivery pen device with dose logging capabilities, this allowing for secure, easy and cost-effective wireless transfer of dose log data from the pen device to e.g. a mobile device such as a smartphone or a tablet computer.

The process of pairing devices in BLE traditionally involves exchanging identification so the two devices know each other in the future. The process can optionally involve exchange of a secret key so that further information exchange can be authenticated (hash) and/or private (encrypted). The pairing process is often a compromise between user convenience and level of security. For example, to avoid man-in-the-middle attacks the pairing process should involve an out of band exchange of keys which in case of BLE typically involves the user having to type in a 6 digit number.

In order to save energy in the data generating device the electronics need to be in a low-power mode (idle) most of the time. A user step is usually required to wake up the device which adds to the inconvenience of pairing.

The present invention utilizes the concept of pairing two devices by tapping them together and detecting the vibration pattern simultaneously in both devices. In an exemplary embodiment the transmitting device is in the form of a drug delivery device provided with dose logging circuitry comprising a piezo sensor, and the receiving external device is in the form of a smartphone.

In the drug delivery device, the vibration pattern is detected by a piezo sensor (either by the same sensor used for dose size determination or by an additional piezo sensor) and in the smartphone by using the microphone, the accelerometer, or other suitable sensors. Alternatively, the drug delivery device may detect a vibration pattern using other detection means, e.g. a microphone, an accelerometer or movement of a galvanic contact.

In an exemplary implementation of the invention the time of the knock is used as a seed to a secret key. That is, both devices records the time of the tap, keeps track of timing of radio communication and uses a (randomized) time elapsed between knock and radio communication as the encryption key seed, see the example below. To prevent that an attacker could listen on the audio signal if close enough, this could be counteracted by the phone emitting false tap sounds.

The concept can be used for a transmit-only device, in which case the transmitting device cannot know if the pairing was successful or if the piezo sensor only detected a random tap (not on the phone). In this case the transmitting device may be provided with a predefined key and tap information combined with a transmitted hint allows the key to be determined in the receiving device. Every time the pen is tapped the hint is changed but the encryption key remains the same.

The achieved key strength for the time only solution depends on the accuracy of the timing, and maximum acceptable delay time, that is, the accuracy of the phone clock, the pen clock, and variability of transfer and reception times. In practice only a few bits of key strength might be expected.

In cases where a stronger key is needed, this idea might be combined with other pairing methods providing part of the encryption key. The method might, for example, enable using a prestored batch key instead of individually coded keys per pen.

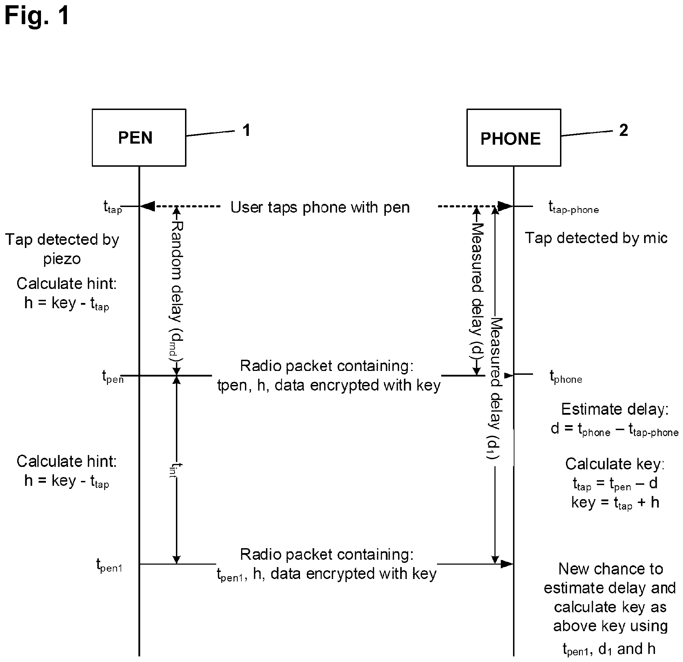

Fig. 1 illustrates an example based on transmit-only communication between a transmitting device (pen device 1) and a receiving storage device (smartphone 2) running an app adapted to receive and process data from the pen device via e.g. Bluetooth® or BLE. In the pen device a tapping event may be used to power up the electronic circuitry and initiate a pairing process, whereas the smartphone will have to be set in a pairing state by the user. The pen device may be provided with a predefined key or a unique key may be generated the first time the pen device detects a pairing impact.

When the pen device and the smartphone are tapped together by the user at a given time, the tapping impact is registered and analysed by the sensor circuitry in each device to determine whether the impact has a pre-defined characteristic indicating that a pairing process is initiated. In case a pairing impact is detected by the pen device a time $t_{tap}$ is registered and stored in the pen device. Correspondingly, if a pairing impact is detected by the pen device a time $t_{tap\_phone}$ is registered and stored in the smartphone.

In the example the pen device calculates a hint h as

$$h = key - t_{tap}.$$

and generates a random time value which is then used as a time delay $d_{rnd}$ for transmitting a radio data packet at time

$$t_{pen} = t_{tap} + d_{rnd}$$

The transmitted radio packet comprises the pen device transmission time $t_{pen}$, the key hint h as well as data encrypted with the key.

When the smartphone receives the radio data packet at time $t_{phone}$ according to the clock the smartphone the time delay $d_{rnd}$ can be estimated as

$$d_{rnd} = t_{phone} - t_{tap\_phone},$$

As appears, in the example the transmission delay between $t_{pen}$ and $t_{phone}$ is disregarded. Using the determined time delay $d_{rnd}$ the pen device tap time can be calculated by the smartphone as

$$t_{tap} = t_{pen} - d_{rnd}$$

and the key as

$$key = t_{tap} + h.$$

As appears, the calculations are based on relative time in each device for which reason it is not necessary that the two clocks are synchronised. Indeed, it is essential for the calculation of $d_{rnd}$ in the smartphone that the "speed" of the two clocks are known, e.g. being the same as in the above example.

The pen might retransmit the packet a number of times to increase probability of reception in bad radio environments, with an updated $t_{pen}$ in each packet.

When a key has been determined by the smartphone, the smartphone will leave the pairing state and the determined key will be used to decrypt data in any subsequently received radio data packet. As the pen device is "blind" in respect of the key having been received by a smartphone, the pen device will transmit the above-described pairing radio packet each time a pairing impact is detected. During normal operation of the pen device, a radio packet comprising encrypted dose log data may be sent at the end of a detected dose event.

If desired the smartphone may be set in the pairing state again and be re-paired with the same pen device, this resulting in the same key being determined.

When the pen device is replaced the smartphone and the new pen device will have to be paired anew resulting in a new key being calculated and stored in the smartphone. In case a radio data packet is received from a non-paired pen device the smartphone will not be able to decrypt the received data which may then be communicated to the user by an error message.

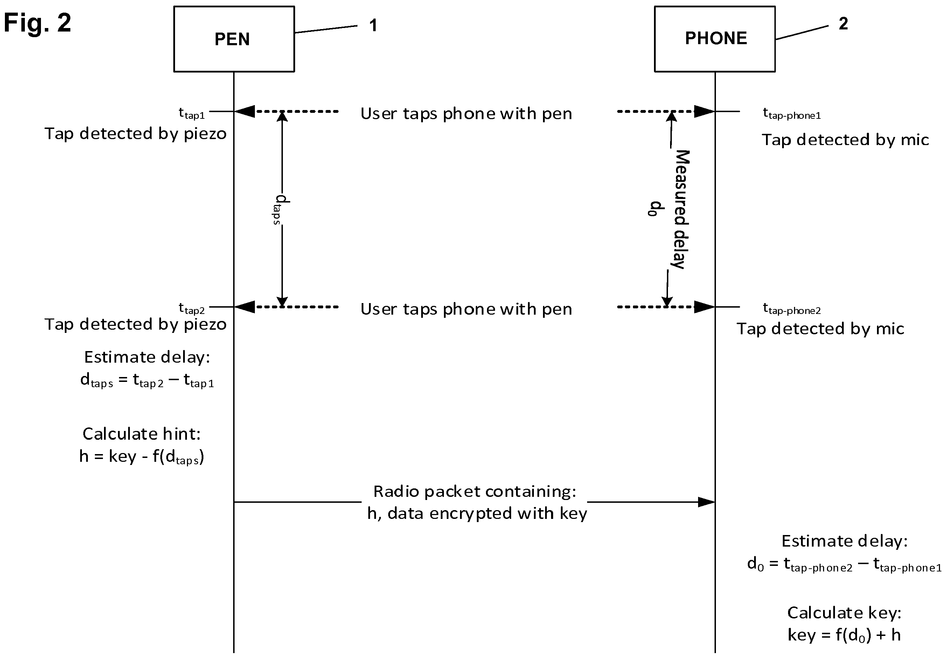

Fig. 2 illustrates a second example based on transmit-only communication between a transmitting pen device and a receiving smartphone in which communication is based on double-tapping the pen and the smartphone against each other.

When the pen device and the smartphone are double-tapped together by the user at a given time, the tapping impacts are registered and analysed by the sensor circuitry in each device in order to determine whether the impacts have the pre-defined characteristic and that the delay between the two taps are within predefined limits, this indicating that a pairing process is initiated.

In case a pairing double-tap event is detected by the pen device times $t_{tap1}$ and $t_{tap2}$ are registered and stored in the pen device. Correspondingly, in the phone $t_{tap\text{-}phone1}$ and $t_{tap\text{-}phone2}$ are registered and stored. The estimated delays are calculated as

$$d_{taps} = t_{tap2} - d_{tap1}$$

$$d_0 = t_{tap\text{-}phone2} - d_{tap\text{-}phone1}$$

As shown in fig. 2 the pen device uses a function f(d) to calculate a hint h as

$$h = key - f(d_{taps})$$

The transmitted radio packet comprises the key hint h as well as data encrypted with the key.

When the smartphone receives the radio data packet the key can be calculated as

$$key = f(d_0) + h.$$

As in the first example the pen may retransmit the packet a number of times to increase probability of reception in bad radio environments.

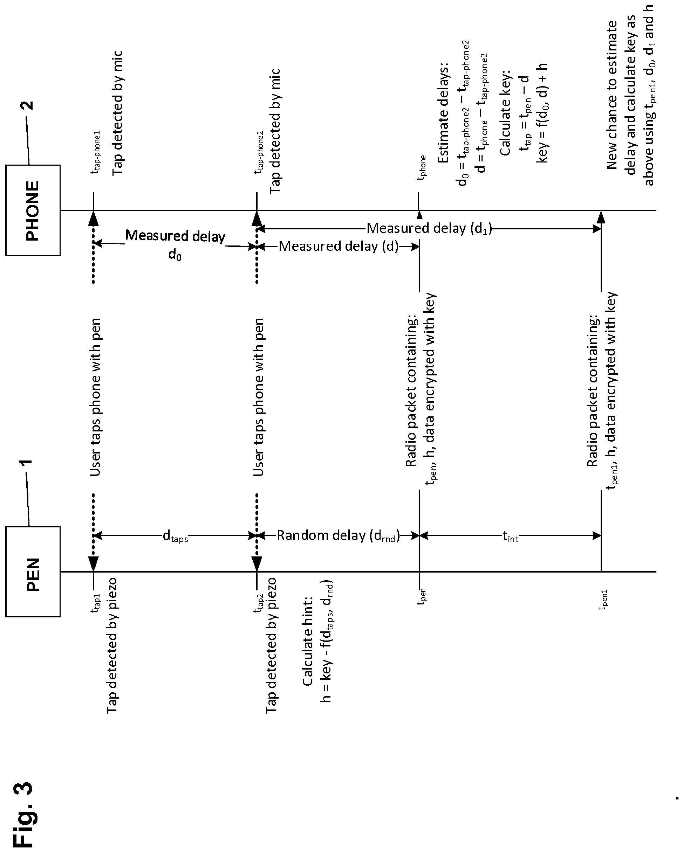

Fig. 3 illustrates a third example based on transmit-only communication between a transmitting pen device and a receiving smartphone in which communication as in the second example is based on double-tapping the pen and the smartphone against each other, however, in addition also a random time value as in the first example is generated.

When the pen device and the smartphone are double-tapped together by the user at a given time, the tapping impacts are registered and analysed by the sensor circuitry in each device in order to determine whether the impacts have the pre-defined characteristic and that the delay between the two taps are within predefined limits, this indicating that a pairing process is initiated.

Correspondingly, when a pairing double-tap event is detected by the pen device a hint h can be calculated as

$$h = key - f(d_{taps}, d_{md})$$

The transmitted radio packet comprises the pen device transmission time $t_{pen}$, the key hint h as well as data encrypted with the key. In the phone delays are estimated as

$$d_0 = t_{tap-phone2} - t_{tap-phone1}$$

$$d = t_{phone} - t_{tap-phone2}$$

And the key calculated as

$$t_{tap} = t_{pen} - d$$

$$key = f(d_0, d) + h$$

The pen may retransmit the packet to increase probability of reception in bad radio environments, with an updated $t_{pen1}$ in each packet and a new $d_1$ measured delay by the phone.

As a further alternative to determine a time related value based on the detected impact event, the "shape" of a detected impact may be used to calculate a pair of corresponding unique time related values in the pen respectively the smartphone.

In the above examples a key for use as an encryption/decryption key has been described, however, the key may be used to code for or identify other properties. For example, the key may denote a drug characteristic such as the strength of a drug formulation, e.g. whether a given pen device comprises a U100 or U200 formulation of insulin, this allowing the received data to be correctly interpreted. The key may also be a combined key allowing a number of keys to be imbedded in a single key value.

After having described an exemplary embodiment of the invention a number of drug delivery devices in which the above-described pairing concept could be implemented will be described.

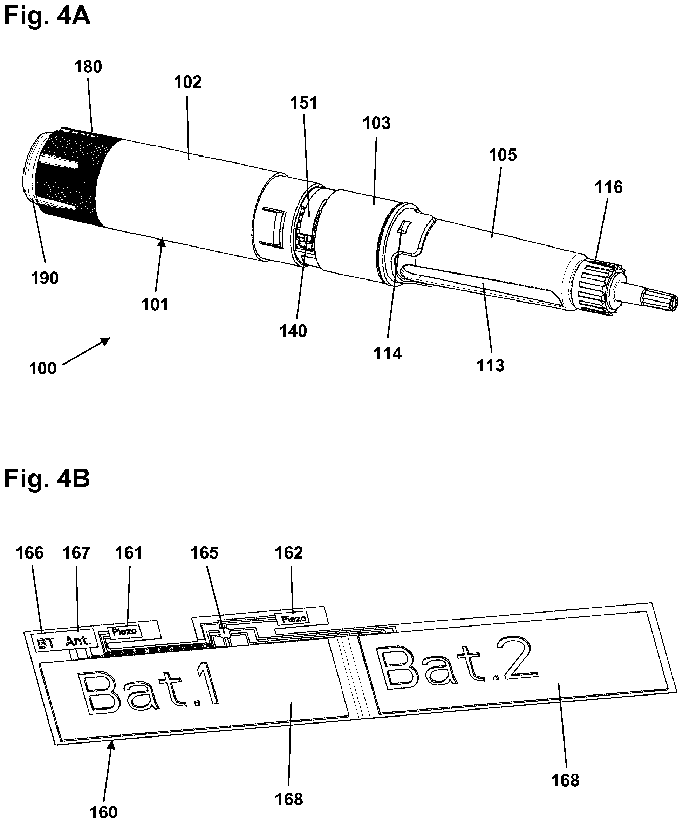

The pen device 100 in fig. 4A comprises a proximal body or drive assembly portion with a housing 101 in which a drug expelling mechanism is arranged or integrated, and a distal cartridge holder portion in which a drug-filled transparent cartridge 113 with a distal needle-penetrable septum is arranged and retained in place by a non-removable cartridge holder attached to the proximal portion. The cartridge holder comprises openings allowing a portion of the cartridge to be inspected, distal coupling means allowing a needle assembly 116 to be releasably mounted as well as proximal coupling means in the form of two opposed protrusions 114 allowing a cap (not shown) to be releasably mounted covering the cartridge holder. In the shown embodiment the housing comprises a proximal housing portion 102 and a distal housing portion 103 which in a fully assembled state of the pen device is fixedly connected to each other via an intermediate tubular housing portion (not shown) covering the shown flexible arm 150 (see below), thereby forming a unitary housing. The cartridge is provided with a piston driven by a piston rod forming part of the expelling mechanism and may for example contain an insulin, GLP-1 or growth hormone formulation. A proximal-most rotatable dose setting member 180 serves to manually set a desired dose of drug and which can then be expelled when the button 190 is actuated. The expelling mechanism comprises a helically rotatable scale drum member with a plurality of indicia in the form of dose size numerals printed thereon, the dose size number corresponding to the currently set dose size being shown in a display opening (not seen in fig. 4A). Depending on the type of expelling mechanism embodied in the drug delivery device, the expelling mechanism may comprise a spring as in the shown embodiment which is strained during dose setting and then released to drive the piston rod when the release button is actuated. Alternatively the expelling mechanism may be fully manual in which case the dose member and the actuation button may be arranged to move proximally during dose setting corresponding to the set dose size, and then to be moved distally by the user to expel the set dose, e.g. as in a FlexPen® manufactured and sold by Novo Nordisk A/S.

Although fig. 3A shows a drug delivery device of the prefilled type, i.e. it is supplied with a premounted cartridge and is to be discarded when the cartridge has been emptied, in alternative embodiments the drug delivery device may be designed to allow a loaded cartridge to be replaced, e.g. in the form of a "rear-loaded" drug delivery device in which the cartridge holder is adapted to be removed from the device main portion, or alternatively in the form of a "front-loaded" device in which a cartridge is inserted through a distal opening in the cartridge holder which is non-removable attached to the main part of the device.

The expelling mechanism incorporated in pen device 100 comprises a ring-formed piston rod drive element and an actuator member 140 in the form of a rotatable component that rotates together with the piston rod drive element during expelling of a dose of drug, the actuator member 140 thereby experiencing unidirectional rotational movement relative to an indicator structure fixedly disposed within the housing 101. In the shown embodiment the indicator structure is in the form of a pair of opposed circumferentially arranged deflectable flexible arms 151 each engaging the actuator member.

The actuator member 140 is in the form of a toothed wheel having a plurality of axially oriented ridges protruding radially outwards and being spaced circumferentially and equidistantly. Each ridge is formed with a gradually rising leading side and a sharply dropping trailing side. In the shown embodiment 24 ridges are spaced with angular steps of 15 degrees. Between any two neighbouring ridges a groove is formed.

Each of the deflectable arms 151 includes at its free end a tip portion with a radially inwards pointing first surface which is angled to be generally parallel with a gradually rising side of a ridge. Each tip portion further has a second opposed surface which is angled to be generally parallel with the sharply dropping side of a ridge. The radially inwards pointing first surface of the tip portions is configured to ride over consecutive ridges as the actuator member 140 rotates relative to the deflectable arms so that the tip portions of the first and second deflectable arm remain in intimate contact with the outer contour of the actuator member 140 as the latter rotates. The free end of a flexible arm 151 is biased slightly inwards when the tip portion is seated in a groove, the biasing force increasing when the free end of the arm is lifted outwards by the ridge formations as the actuator member rotates.

In the shown embodiment, the tip portions of the deflectable arms are located approximately 178 degrees apart so that, as the actuator member 140 rotates, the first deflectable arm will experience cooperation with a particular first ridge slightly before the second deflectable arm will experience cooperation with a ridge arranged diametrically opposite from the first protrusion. This arrangement is described in greater detail in EP application 17205309.

Alternatively, a single arm design may be used.

In order to monitor operation of the device by electronic means, electronic circuitry 160 is disposed in or on the device 100 for registering events associated with operations performed by the device, i.e. expelling of a set dose of drug. In the shown embodiment of fig. 4B the electronic circuitry 160 is in the form of a flexible sheet on which is formed and mounted input means adapted to be actuated, directly or indirectly, by movement of the indicator structure(s), a processor and memory 165, wireless communication means 166 with antenna 167, and an energy source 168, wherein the processor is adapted to determine on the basis of measured values from the input means a rotational position and/or a rotational movement of the actuator member 140 to thereby calculate the size of an expelled dose of drug. The flexible sheet is adapted to be mounted on housing parts of the pen device by e.g. adhesive means, the nature of the flexible sheet allowing it to be mounted also on curved surfaces.

In the shown embodiment the input means is active transducers in the form of piezoelectric sensors 161, 162 adapted to be mounted onto the flexible arms 151 and thereby generating an output as the flexible arms are moved by the rotating actuator member 140. Although not incorporated in the shown embodiment, the electronic circuitry may in other embodiments further include a display so as to offer a visible read-out of information related to registered events. In the shown embodiment energy is provided by two electric cells 168.

One or more of the above-described components may be printed onto the flexible sheet, e.g. the piezoelectric sensors, a display, the antenna and the energy source. Other components, e.g. the processor and associated memory as well as a BLE radio chip may be surface mounted on the flexible sheet.

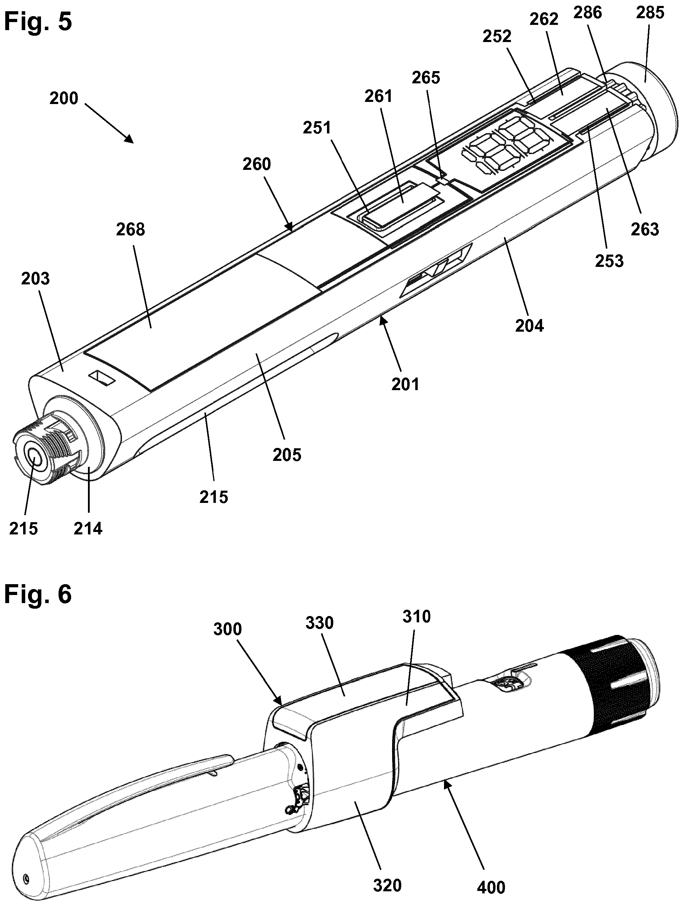

Turning to fig. 5 a further pen device 200 incorporating electronic circuitry for the generation of a dynamic dose log will be described. The pen device 100 of fig. 4A could be considered a traditional drug delivery device provided with electronic circuitry for the creation and transmission of a dose log, the pen device having a traditional user interface and being operated by a user in a traditional way, i.e. setting a dose size while observing a mechanical scale drum. In contrast, the pen device 200 is provided with a digital display replacing the traditional scale drum.

More specifically, the pen device 200 comprises a cylindrical housing 201 having a slightly curved information display surface 203 and a more conventionally curved opposing surface 204. The device is shown without a covering foil label, this allowing the electronic circuitry to be seen. The housing accommodates a drug containing cartridge 213, which has been inserted through an opening at a distal end thereof. The cartridge, which is closed at its distal end by a penetrable self-sealing septum 215 and at its proximal end by a slidable piston (not visible), is arranged in the distal cartridge holder portion 205 of the housing, being snapped to a proximal interior surface of the housing 201 by a snap coupling formed as part of the cartridge needle mount member 214 serving as an attachment interface for an injection needle unit (not shown). The housing is provided with a longitudinal window 206 for inspection of the cartridge contents and further accommodates both a dose setting mechanism and a drug expelling mechanism. The dose setting and expelling mechanism may be of any suitable design, e.g. a spring-driven design as shown, albeit without a scale drum. In the shown embodiment dose setting and dose release is performed using a combined dose setting and dose release member 285, i.e. the combined member is adapted to both rotate relative to the housing 201 during dose setting and to be moved axially to release a set dose.

As in the above-described embodiment, the expelling mechanism comprises an actuator member in the form of a rotatable component that rotates together with the piston rod drive element during expelling of a dose of drug, the actuator member thereby experiencing unidirectional rotational movement relative to an indicator structure fixedly disposed within the housing 201. In the shown embodiment the indicator structure is in the form of an axially arranged deflectable flexible arm 150 engaging the actuator member.

The combined dose setting and release member 285 extends into the housing 201 from a proximal end thereof. The combined member 285 comprises a cylindrical main body which is rotatable about a longitudinal axis of the housing. An axially grooved smaller-diameter actuator collar 286 is provided just distally of the main body and extends into the housing. The grooves have a spacing of 15 degrees and serve as actuators for dose setting input means, each groove corresponding to an increment of one dose unit, i.e. typically 1 IU of insulin.

In the housing 201 central portion some wall material has been removed to provide the above-mentioned radially deflectable flexible dose expelling arm 250, and in a proximal portion wall material has been removed to provide first and second radially deflectable dose setting arms 251, 252, the latter being actuated by the grooved actuator collar 286. As described in greater detail in WO 2018/078178 the two dose setting arms allow incremental up/down rotation of the combined member 285 to be determined, this in turn being used to control the display to show the presently set dose size.

In order to monitor operation of the device by electronic means, electronic circuitry 260 is disposed on the device 200 for registering events associated with operations performed by the device, i.e. expelling of a set dose of drug. In the shown embodiment the electronic circuitry 260 is in the form of a flexible sheet on which is formed and mounted input means adapted to be actuated by movement of the indicator structures 250, 251, 252, a processor with memory and wireless communication means 265, a display 269 and an energy source 268, wherein the processor is adapted to determine on the basis of measured values from the input means a rotational position and/or a rotational movement of the actuator member to thereby calculate the size of an expelled dose of drug. The flexible sheet is adapted to be mounted on the curved housing surface 203 of the pen device by e.g. adhesive means.

In the shown embodiment the input means is active transducers in the form of piezoelectric sensors 261, 262, 263 adapted to be mounted onto the flexible arms 251, 252, 253 and thereby generating an output as the flexible arms are moved by the rotating actuator member respectively the dose setting actuator collar 286.

One or more of the above-described components may be printed onto the flexible sheet, e.g. the piezoelectric sensors, the display, an antenna and the energy source in the form of an electric cell. Other components, e.g. the processor and associated memory as well as a BLE radio chip may be surface mounted on the flexible sheet.

A further type of a drug delivery device comprising integrated dose logging circuitry is in the form of a traditional manual (i.e. non- spring-driven) drug delivery device in which the dose setting and actuation button will extend axially from the device as a dose is being set, the dose logging circuitry being arranged in the dose setting button and comprising e.g. a traditional rotary encoder adapted to register rotation during dose setting and/or dose expelling. A specific example of such a device is sold and manufactured by Novo Nordisk A/S as the Novo-Pen® 6, a pen device provided with wireless communication means allowing dose log data to be transferred to an external device using the above-described transmission protocol. NovoPen® 6 is provided with a display, however, this feature could alternatively be dispensed with.

A further example of how a drug delivery device can be provided with dose logging circuitry is disclosed in WO 2014/128155, relating to an electronic logging unit adapted to be housed in a drug-filled cartridge having an axially displaceable piston and an outer cavity formed between the piston and the cartridge proximal opening, the logging unit comprising a general axis, a first distal portion adapted to engage the cartridge piston, and a second proximal portion adapted to engage a rotating element having a rotational axis corresponding to the general axis. The unit is provided with electronic circuitry and sensor means adapted to detect the amount of relative rotation between the first and second portions, storage means adapted to store data representing detected amounts of relative rotation, as well as transmitter means allowing the data to be transmitted to an external device. By this arrangement a logging unit can be provided in an essentially un-modified drug delivery device comprising a piston rod rotating during dose delivery, the rotation being transferred to the proximal portion of the logging unit, the latter being rotationally locked to the cartridge piston. This said, it may be necessary to use a cartridge with a more distally arranged piston to make room for the logging unit. The logging unit may be provided as an "add-on" allowing a conventional durable, i.e. re-usable, drug delivery device to be provided with a logging functionality when needed. For example, when initiating a given patient on an insulin regimen the prescribing doctor may provide the patient with a drug delivery device in which a logging unit has been inserted, this allowing the doctor to check to which degree the patient has been in compliance with the regiment when the device is returned to the doctor after use. Indeed, the same logging unit could be used on a regular basis by any patient for which the logging capability and user interface are desirable. Alternatively, the logging unit could be provided in a disposable, prefilled device.

The electronic logging unit disclosed in WO 2014/128155 utilizes a rotary sensor based on galvanic contacts. To provide an impact sensor the logging unit may be provided with additional means for this purpose, e.g. a piezo sensor, an accelerometer or a microphone. Alternatively, the impact sensor may be based on the galvanic contacts in the rotary sensor *per se.* As these may be sensitive to vibration the signal from the sensor may be filtered by a hardware or software based algorithm to not be interpreted as a dosing event. Correspondingly, in the same way a given impact event could be detected.

Turning to fig. 6 an add-on dose logging device 300 mounted on a drug delivery pen device 400 of the spring-driven type is shown, the add-on device incorporating electronic circuitry for the generation of a dynamic dose log when mounted on the pen device. In the present context the device represents a "generic" drug delivery device providing a specific example of a device in combination with which embodiments of the present invention can be used.

The logging module 300 comprises a body portion 310 and a ring-formed portion 320 allowing the add-on device to be mounted on a generally cylindrical pen device. The body portion comprises electronic circuitry and sensor means allowing a property to be detected representing an amount of drug being expelled from the cartridge, as well as an optional display 330 for displaying data to a user. The ring portion comprises coupling means allowing the add-on device to be securely and correctly mounted on the pen body. The electronic circuitry and the sensor means may in part be arranged in the ring portion.

The pen device comprises an indicator element with a magnet rotating together therewith during expelling of a dose of drug, the magnet being configured to generate a spatial magnetic field which relative to the sensor means varies corresponding to the spatial position and orientation of the magnet. The add-on device comprises sensor means adapted to measure a magnetic field as well as processor means configured to determine on the basis of measured values rotational movement and/or positions of the indicator element on the basis of which a dose log can be created. An exemplary embodiment of both the add-on device and the pen device is described in greater detail in WO 2014/161952.

Additionally, the shown add-on device 300 can be provided with wireless communication means allowing dose log data to be transferred to an external device using the above-described transmission protocol.

A further example of an add-on dose logging device adapted to be mounted on a drug delivery pen device of the spring-driven type, and which may incorporate aspects of the present invention, is shown in PCT/EP2018/075639.

In the above description of exemplary embodiments, the different structures and means providing the described functionality for the different components have been described to a degree to which the concept of the present invention will be apparent to the skilled reader.

The detailed construction and specification for the different components are considered the object of a normal design procedure performed by the skilled person along the lines set out in the present specification.

**Claims**

1. A computer-implemented method of wireless pairing of two devices for subsequent secure transmission of data, comprising the steps of:

   - providing a transmitter device (1, 100, 200, 300) adapted to wirelessly transmit data, comprising:

     - transmitter impact sensor circuitry for detecting an impact event made to the transmitter device, and
     - a transmitter clock,

   wherein, when an impact event having a pre-defined characteristic is detected by the transmitter impact sensor circuitry, the transmitter device is adapted to:

     - determine at least one unique time related value based on the detected impact event, and
     - initiate a wireless pairing process,
     - providing a receiver device (2) adapted to wirelessly receive data transmitted from the transmitter device (1, 100, 200, 300), comprising:

       - receiver impact sensor circuitry for detecting an impact event made to the receiver device, and
       - a receiver clock,

   wherein, when an impact event having a pre-defined characteristic is detected by the receiver impact sensor circuitry, the receiver device is adapted to:

     - determine at least one corresponding time related value based on the detected impact event, and
     - initiate a wireless pairing process,
     - impacting the transmitter device and the receiver device against each other,

   wherein the transmitter device pairing process comprises the steps of:

     • retrieving or determining a key K for coding and decoding data,
     • based on the key K and at least one unique time related value, calculating a hint H, and
     • transmitting a data package comprising the hint H and including at least one unique time related value,

   wherein the receiver device pairing process comprises the steps of:

     • receiving the data package transmitted from the transmitter device, and

• based on the hint H and at least one corresponding time related value, calculating and storing the key K.

2. A computer-implemented method of wireless pairing as in claim 1, wherein:

- a determined unique time related value is a unique time stamp T1 and a determined corresponding time related value is a corresponding time stamp T2,
- the transmitter device pairing process comprises the further steps of:

• starting a transmitter timer,
• determining a time delay value D, and
• after a time delay of D from start of the transmitter timer, transmitting the data package comprising the value (T1+D) and hint H,

- the receiver device pairing process comprises the further steps of:

• starting a receiver timer,
• determining the time T when the data package is received,
• estimating D based on the difference between the time T and T2,
• calculating T1 as the difference between (T1+D) and the estimated D, and
• based on the hint H and the value T1, calculating and storing the key K.

3. A computer-implemented method of wireless pairing as in claim 1, wherein:

- the impact event comprises at least two impacts on the basis of which a unique time related value V1 and a corresponding time related value V2 are determined,
- the hint H is calculated based on the unique time related value V1, and
- the key K is calculated by the receiver device based on the hint H and the corresponding time related value V2.

4. A computer-implemented method of wireless pairing as in claim 1, wherein

- a unique time related value V1 and a corresponding time related value V2 are determined based on the waveforms of the detected impacts,
- the hint H is calculated based on the unique time related value V1, and
- the key K is calculated by the receiver device based on the hint H and the corresponding time related value V2.

5. A computer-implemented method of wireless pairing as in claim 3 or 4, wherein:

- a unique time stamp T1 and a corresponding time stamp T2 are determined based on the detected impact events,
- the transmitter device pairing process comprises the further steps of:

• starting a transmitter timer,
• determining a time delay value D,
• calculating the hint H based on D and the unique time related value V1, and
• after a time delay of D from start of the transmitter timer, transmitting the data package comprising the value (T1+D) and hint H,

- the receiver device pairing process comprises the further steps of:

• starting a receiver timer,
• determining the time T when the data package is received,
• estimating D based on the difference between the time T and T2,
• calculating T1 as the difference between (T1+D) and the estimated D, and
• based on the hint H, the corresponding time related value V2 and the value T1, calculating and storing the key K.

6. A computer-implemented method of wireless pairing as in any of claims 1-5, wherein the transmitter device pairing process comprises the transmission of data encrypted with the key K.

7. A computer-implemented method of wireless pairing as in any of claims 1-6, wherein data transmitted from the transmitter device is always encrypted using the key K.

8. A computer-implemented method of wireless pairing as in any of claims 1-7, wherein the transmitter device will initiate a wireless pairing process each time an impact having a pre-defined characteristic is detected by the transmitter impact sensor circuitry.

9. A computer-implemented method of wireless pairing as in any of claims 1-8, wherein the receiver device will initiate a wireless pairing process only when in a pairing state.

10. A computer-implemented method of wireless pairing as in any of claims 1-9, wherein:

- the transmitter impact sensor circuitry comprises a piezo sensor and the receiver impact sensor circuitry comprises a microphone or an accelerometer.

11. A computer-implemented method of wireless pairing as in any of claims 1-10, wherein:

- the receiver device is operational between (i) a non-pairing state, and (ii) a pairing state in which an impact having the pre-defined characteristic can be detected and will result in the receiver device performing the receiver device pairing process.

12. A transmitter device (1, 100, 200, 300), comprising:

- electronic circuitry comprising:

- wireless transmission means for wireless transfer of data to an external device,
- impact sensor circuitry for detecting an impact made to the transmitter device, and
- a clock,

wherein, when an impact to the transmitter device having a pre-defined characteristic is detected by the impact sensor circuitry, the transmitter device is adapted to:

- determine at least one time related value based on the detected impact event, and
- initiate a wireless pairing process comprising the steps of:

• retrieving or determining a key K for coding and decoding data,
• based on the key K and at least one time related value, calculating a hint H,
• transmitting a data package comprising the hint H and including at least one time related value.

13. A transmitter device as in claim 14, the transmitter device being in the form of a drug delivery device (100, 200) or a drug delivery assembly (300, 400), further comprising:

- a drug reservoir or means for receiving a drug reservoir, and
- drug expelling means comprising dose setting means allowing a user to set a dose amount of drug to be expelled,

the electronic circuitry further comprising:

- sensor means adapted to capture a property value related to the dose amount of drug expelled from a reservoir by the expelling means during an expelling event, and
- storage means adapted to store data in the form of a plurality of property values to thereby create a log.

14. A receiver device (2), comprising:

- electronic circuitry comprising:

- wireless data receiving means,
- impact sensor circuitry for detecting an impact made to the receiver device,
- a clock, and
- a stored algorithm for calculating a key K based on a received hint H and an impact related time value,

wherein, when an impact to the receiver device having a pre-defined characteristic is detected by the impact sensor circuitry, the receiver device is adapted to:

- determine at least one time related value based on the detected impact event, and
- initiate a wireless pairing process comprising the steps of:

• receiving a data package comprising a hint H,
• based on a received hint H and at least one determined time related value, calculating and storing the key K.

15. A receiver device as in claim 14, the receiver device being in the form of a storage device further comprising:

- storage means adapted to store data in the form of a plurality of property values related to dose amounts of drug to thereby create a dose log.

**Patentansprüche**

1. Computerimplementiertes Verfahren zur drahtlosen Kopplung von zwei Vorrichtungen zur anschließenden sicheren Übertragung von Daten, umfassend die Schritte:

- Bereitstellen einer zur drahtlosen Übertragung von Daten geeigneten Sendervorrichtung (1, 100, 200, 300), umfassend:

- Sender-Einwirkungssensor-Schaltung zur Erkennung eines Einwirkungsereignisses auf die Sendervorrichtung, und

- einen Sender-Zeitgeber,

wobei, wenn ein Einwirkungsereignis mit einer vordefinierten Charakteristik durch die Sender-Einwirkungssensor-Schaltung erfasst wird, die Sendervorrichtung ausgelegt ist zum:

- Ermitteln zumindest eines eindeutigen zeitbezogenen Werts basierend auf dem erkannten Einwirkungsereignis, und
- Einleiten eines drahtlosen Kopplungsprozesses,
- Bereitstellen einer Empfängervorrichtung (2), die zum drahtlosen Empfangen von Daten ausgelegt ist, die von der Sendervorrichtung (1, 100, 200, 300) übertragen werden, umfassend:

- Empfänger-Einwirkungssensor-Schaltung zur Erkennung eines Einwirkungsereignisses auf die Empfängervorrichtung, und
- einen Empfänger-Zeitgeber,

wobei, wenn ein Einwirkungsereignis mit einer vordefinierten Charakteristik durch die Empfänger-Einwirkungssensor-Schaltung erfasst wird, die Empfängervorrichtung ausgelegt ist zum:

- Ermitteln zumindest eines entsprechenden zeitbezogenen Werts basierend auf dem erkannten Einwirkungsereignis, und
- Einleiten eines drahtlosen Kopplungsprozesses,
- Einwirken der Sendervorrichtung und der Empfängervorrichtung gegeneinander, wobei der Kopplungsprozess der Sendervorrichtung die folgenden Schritte umfasst:

• Abrufen oder Bestimmen eines Schlüssels K zum Kodieren und Dekodieren von Daten,
• basierend auf dem Schlüssel K und zumindest einem eindeutigen zeitbezogenen Wert, Berechnen eines Hinweises H, und
• Übertragen eines Datenpakets, das den Hinweis H und zumindest einen eindeutigen zeitbezogenen Wert enthält,

wobei der Kopplungsprozess des Empfängergeräts die folgenden Schritte umfasst:

• Empfangen des von der Sendervorrichtung übertragenen Datenpakets, und
• Berechnen und Speichern des Schlüssels K basierend auf dem Hinweis H und zumindest einem entsprechenden zeitbezogenen Wert.

2. Computerimplementiertes Verfahren zur drahtlosen Kopplung nach Anspruch 1, wobei:

- ein ermittelter eindeutiger zeitbezogener Wert ein eindeutiger Zeitstempel T1 ist und ein ermittelter entsprechender zeitbezogener Wert ein entsprechender Zeitstempel T2 ist,
- der Kopplungsprozess der Sendervorrichtung die folgenden weiteren Schritte umfasst:

• Starten eines Sender-Zeitgebers,
• Ermitteln eines Zeitverzögerungswertes D, und
• nach einer Zeitverzögerung von D ab dem Start des Sender-Zeitgebers, Übertragen des Datenpakets mit dem Wert (T1+D) und dem Hinweis H,

- der Kopplungsprozess der Empfängervorrichtung die folgenden weiteren Schritte umfasst:

• Starten eines Empfänger-Zeitgebers,
• Ermitteln des Zeitpunkts T, zu dem das Datenpaket empfangen wird,
• Schätzen von D basierend auf der Differenz zwischen der Zeit T und T2,
• Berechnen von T1 als die Differenz zwischen (T1+D) und dem geschätzten D, und
• Berechnen und Speichern des Schlüssels K basierend auf dem Hinweis H und dem Wert T1.

3. Computerimplementiertes Verfahren zur drahtlosen Kopplung nach Anspruch 1, wobei:

- das Einwirkungsereignis zumindest zwei Einwirkungen umfasst, auf deren Grundlage ein eindeutiger zeitbezogener Wert V1 und ein entsprechender zeitbezogener Wert V2 ermittelt werden,
- der Hinweis H basierend auf dem eindeutigen zeitbezogenen Wert V1 berechnet wird, und
- der Schlüssel K von der Empfängervorrichtung basierend auf dem Hinweis H und dem entsprechenden zeitbezogenen Wert V2 berechnet wird.

4. Computerimplementiertes Verfahren zur drahtlosen Kopplung nach Anspruch 1, wobei

- ein eindeutiger zeitbezogener Wert V1 und ein entsprechender zeitbezogener Wert V2 basierend auf den Wellenformen der erkannten Einwirkungen ermittelt werden,
- der Hinweis H basierend auf dem eindeutigen zeitbezogenen Wert V1 berechnet wird, und
- der Schlüssel K von der Empfängervorrichtung basierend auf dem Hinweis H und dem entsprechenden zeitbezogenen Wert V2 berechnet wird.

**5.** Computerimplementiertes Verfahren zur drahtlosen Kopplung nach Anspruch 3 oder 4, wobei:

- ein eindeutiger Zeitstempel T1 und ein entsprechender Zeitstempel T2 basierend auf den erkannten Einwirkungsereignissen ermittelt werden,
- der Kopplungsprozess der Sendervorrichtung die folgenden weiteren Schritte umfasst:

  • Starten eines Sender-Zeitgebers,
  • Ermitteln eines Zeitverzögerungswertes D,
  • Berechnen des Hinweises H basierend auf D und dem eindeutigen zeitbezogenen Wert V1, und
  • nach einer Zeitverzögerung von D ab dem Start des Sender-Zeitgebers, Übertragen des Datenpakets mit dem Wert (T1+D) und dem Hinweis H,

- der Kopplungsprozess der Empfängervorrichtung die folgenden weiteren Schritte umfasst:

  • Starten eines Empfänger-Zeitgebers,
  • Ermitteln des Zeitpunkts T, zu dem das Datenpaket empfangen wird,
  • Schätzen von D basierend auf der Differenz zwischen der Zeit T und T2,
  • Berechnen von T1 als die Differenz zwischen (T1+D) und dem geschätzten D, und
  • Berechnen und Speichern des Schlüssels K basierend auf dem Hinweis H, dem entsprechenden zeitbezogenen Wert V2 und dem Wert T1.

**6.** Computerimplementiertes Verfahren zur drahtlosen Kopplung nach einem der Ansprüche 1 bis 5, wobei der Kopplungsprozess der Sendervorrichtung die Übertragung von mit dem Schlüssel K verschlüsselten Daten umfasst.

**7.** Computerimplementiertes Verfahren zur drahtlosen Kopplung nach einem der Ansprüche 1 bis 6, wobei die von der Sendervorrichtung übertragenen Daten immer mit dem Schlüssel K verschlüsselt werden.

**8.** Computerimplementiertes Verfahren zur drahtlosen Kopplung nach einem der Ansprüche 1 bis 7, wobei die Sendervorrichtung jedes Mal einen drahtlosen Kopplungsprozess einleitet, wenn eine Einwirkung mit einer vordefinierten Charakteristik von der Sender-Einwirkungssensor-Schaltung erkannt wird.

**9.** Computerimplementiertes Verfahren zur drahtlosen Kopplung nach einem der Ansprüche 1 bis 8, wobei die Empfängervorrichtung einen drahtlosen Kopplungsprozess nur dann einleitet, wenn sie sich in einem Kopplungszustand befindet.

**10.** Computerimplementiertes Verfahren zur drahtlosen Kopplung nach einem der Ansprüche 1 bis 9, wobei:

- die Sender-Einwirkungssensor-Schaltung einen Piezo-Sensor umfasst und die Emfänger-Einwirkungssensor-Schaltung ein Mikrofon oder einen Beschleunigungsmesser umfasst.

**11.** Computerimplementiertes Verfahren zur drahtlosen Kopplung nach einem der Ansprüche 1 bis 10, wobei:

- die Empfängervorrichtung zwischen (i) einem Nichtkopplungszustand und (ii) einem Kopplungszustand betriebsbereit ist, in dem eine Einwirkung mit der vordefinierten Charakteristik erkannt werden kann und dazu führt, dass die Empfängervorrichtung den Kopplungsprozess der Empfängervorrichtung durchführt.

**12.** Sendervorrichtung (1, 100, 200, 300), umfassend:

- elektronische Schaltung, umfassend:

  - drahtloses Übertragungsmittel zur drahtlosen Datenübertragung an eine externe Vorrichtung,
  - Einwirkungssensor-Schaltungen zur Erkennung einer Einwirkung auf die Sendervorrichtung und
  - eine Uhr,

wobei die Sendervorrichtung, wenn eine Einwirkung auf die Sendervorrichtung mit einer vordefinierten Charakteristik von der Einwirkungssensor-Schaltung erkannt wird, ausgelegt ist zum:

- Ermitteln zumindest eines zeitbezogenen Werts basierend auf dem erkannten Einwirkungsereignis, und
- Einleiten eines drahtlosen Kopplungsprozesses, umfassend die Schritte:

  • Abrufen oder Bestimmen eines Schlüssels K zum Kodieren und Dekodieren von Daten,
  • basierend auf dem Schlüssel K und zumindest einem zeitbezogenen Wert, Berechnen eines Hinweises H,
  • Übertragen eines Datenpakets, das den Hinweis H und zumindest einen zeitbezogenen Wert enthält.

**13.** Übertragungsvorrichtung nach Anspruch 14, wobei die Übertragungsvorrichtung in Form einer Arzneimittelabgabevorrichtung (100, 200) oder einer Arzneimittelabgabeanordnung (300, 400) vorliegt, ferner umfassend:

- einen Arzneimittelvorratsbehälter oder Mittel zur Aufnahme eines Arzneimittelvorratsbehälters, und
- Mittel zum Ausstoßen von Arzneimitteln, umfassend Mittel zur Dosiseinstellung, die es einem Benutzer ermöglichen, eine Dosismenge des auszustoßenden Arzneimittels einzustellen,

wobei die elektronische Schaltung ferner umfasst:

- Sensormittel, die für das Erfassen eines Eigenschaftswerts ausgelegt sind, der sich auf die Dosismenge des Arzneimittels bezieht, die von den Ausstoßmitteln während eines Ausstoßvorgangs aus einem Vorratsbehälter ausgestoßen wird, und
- Speichermittel, die zum Speichern von Daten in Form einer Vielzahl von Eigenschaftswerten ausgelegt sind, um dadurch ein Protokoll zu erstellen.

14. Empfängervorrichtung (2), umfassend:

- elektronische Schaltung, umfassend:

- Mittel zum drahtlosen Datenempfang,
- Einwirkungssensor-Schaltung zur Erkennung einer Einwirkung auf die Empängervorrichtung,
- eine Uhr, und
- einen gespeicherten Algorithmus zur Berechnung eines Schlüssels K basierend auf einem empfangenen Hinweis H und einem einwirkungsbezogenen Zeitwert,

wobei, wenn eine Einwirkung auf die Empfängervorrichtung mit einer vordefinierten Charakteristik durch die Einwirkungssensor-Schaltung erfasst wird, die Empfängervorrichtung ausgelegt ist zum:

- Ermitteln zumindest eines zeitbezogenen Werts basierend auf dem erkannten Einwirkungsereignis, und
- Einleiten eines drahtlosen Kopplungsprozesses, umfassend die Schritte:

• Empfangen eines Datenpakets umfassend einen Hinweis H,
• Berechnen und Speichern des Schlüssels K basierend auf einem empfangenen Hinweis H und zumindest einem ermittelten zeitbezogenen Wert.

15. Empfängervorrichtung nach Anspruch 14, wobei die Empfängervorrichtung in Form einer Speichervorrichtung vorliegt, ferner umfassend:

- Speichermittel, die zum Speichern von Daten in Form einer Vielzahl von Eigenschaftswerten ausgelegt sind, die sich auf Dosismengen von Arzneimitteln beziehen, um dadurch ein Dosisprotokoll zu erstellen.

## Revendications

1. Procédé mis en œuvre par ordinateur d'appairage sans fil de deux dispositifs pour une transmission sécurisée ultérieure de données, comprenant les étapes de :

- fourniture d'un dispositif émetteur (1, 100, 200, 300) adapté à la transmission de données sans fil, comprenant :

- des circuits de capteur d'impact d'émetteur destinés à détecter un événement d'impact exercé sur le dispositif émetteur, et
- une horloge d'émetteur,

dans lequel, lorsqu'un événement d'impact ayant une caractéristique prédéfinie est détecté par les circuits de capteur d'impact d'émetteur, le dispositif émetteur est adapté :

- à la détermination d'au moins une valeur unique liée au temps sur la base de l'événement d'impact détecté, et
- au lancement d'un processus d'appairage sans fil,
- fourniture d'un dispositif récepteur (2) adapté à la réception sans fil de données transmises depuis le dispositif émetteur (1, 100, 200, 300), comprenant :

- des circuits de capteur d'impact de récepteur destinés à détecter un événement d'impact exercé sur le dispositif récepteur, et
- une horloge de récepteur,

dans lequel, lorsqu'un événement d'impact ayant une caractéristique prédéfinie est détecté par les circuits de capteur d'impact de récepteur, le dispositif récepteur est adapté :

- à la détermination d'au moins une valeur correspondante liée au temps sur la base de l'événement d'impact détecté, et
- au lancement d'un processus d'appairage sans fil,
- création d'un impact du dispositif émetteur et du dispositif récepteur l'un contre l'autre,

dans lequel le processus d'appairage de dispositif émetteur comprend les étapes de :

• récupération ou détermination d'une clé K pour le codage et le décodage de données,
• sur la base de la clé K et d'au moins une valeur unique liée au temps, calcul d'un indice H, et
• transmission d'un paquet de données comprenant l'indice H et comportant au moins une valeur unique liée au temps,

dans lequel le processus d'appairage de dispositif récepteur comprend les étapes de :

• réception du paquet de données transmis depuis le dispositif émetteur, et
• sur la base de l'indice H et d'au moins une valeur correspondante liée au temps, calcul et stockage de la clé K.

2. Procédé mis en oeuvre par ordinateur d'appairage sans fil selon la revendication 1, dans lequel :

- une valeur unique liée au temps déterminée est une estampille temporelle unique T1 et une valeur correspondante liée au temps déterminée est une estampille temporelle correspondante T2,
- le processus d'appairage de dispositif émetteur comprend les étapes supplémentaires de :

• démarrage d'une minuterie d'émetteur,
• détermination d'une valeur de retard temporel D, et
• après un retard temporel de D à partir du début de la minuterie d'émetteur, transmission du paquet de données comprenant la valeur (T1+D) et l'indice H,

- le processus d'appairage de dispositif récepteur comprend les étapes supplémentaires de :

• démarrage d'une minuterie de récepteur,
• détermination du temps T auquel le paquet de données est reçu,
• estimation de D sur la base de la différence entre le temps T et T2,
• calcul de T1 en tant que différence entre (T1+D) et le D estimé, et
• sur la base de l'indice H et de la valeur T1, calcul et stockage de la clé K.

3. Procédé mis en oeuvre par ordinateur d'appairage sans fil selon la revendication 1, dans lequel :

- l'événement d'impact comprend au moins deux impacts sur la base desquels une valeur unique liée au temps V1 et une valeur correspondante liée au temps V2 sont déterminées,
- l'indice H est calculé sur la base de la valeur unique liée au temps V1, et
- la clé K est calculée par le dispositif récepteur sur la base de l'indice H et de la valeur correspondante liée au temps V2.

4. Procédé mis en oeuvre par ordinateur d'appairage sans fil selon la revendication 1, dans lequel

- une valeur unique liée au temps V1 et une valeur correspondante liée au temps V2 sont déterminées sur la base des formes d'onde des impacts détectés,
- l'indice H est calculé sur la base de la valeur unique liée au temps V1, et
- la clé K est calculée par le dispositif récepteur sur la base de l'indice H et de la valeur correspondante liée au temps V2.

5. Procédé mis en oeuvre par ordinateur d'appairage sans fil selon la revendication 3 ou 4, dans lequel :

- une estampille temporelle unique T1 et une estampille temporelle correspondante T2 sont déterminées sur la base des événements d'impact détectés,
- le processus d'appairage de dispositif émetteur comprend les étapes supplémentaires de :

• démarrage d'une minuterie d'émetteur,
• détermination d'une valeur de retard temporel D,
• calcul de l'indice H sur la base de D et de la valeur unique liée au temps V1, et
• après un retard temporel de D à partir du début de la minuterie d'émetteur, transmission du paquet de données comprenant la valeur (T1+D) et l'indice H,

- le processus d'appairage de dispositif récepteur comprend les étapes supplémentaires de :

• démarrage d'une minuterie de récepteur,
• détermination du temps T auquel le paquet de données est reçu,
• estimation de D sur la base de la différence entre le temps T et T2,
• calcul de T1 en tant que différence entre (T1+D) et le D estimé, et
• sur la base de l'indice H, de la valeur correspondante liée au temps V2 et de la valeur T1, calcul et stockage de la clé K.

6. Procédé mis en oeuvre par ordinateur d'appairage sans fil selon l'une quelconque des revendications 1 à 5, dans lequel le processus d'appairage de dispositif émetteur comprend la transmission de données chiffrées avec la clé K.

7. Procédé mis en œuvre par ordinateur d'appairage sans fil selon l'une quelconque des revendications 1 à 6, dans lequel les données transmises depuis le dispositif émetteur sont toujours chiffrées à l'aide de la clé K.

8. Procédé mis en œuvre par ordinateur d'appairage sans fil selon l'une quelconque des revendications 1 à 7, dans lequel le dispositif émetteur lance un processus d'appairage sans fil chaque fois qu'un impact ayant une caractéristique prédéfinie est détecté par les circuits de capteur d'impact d'émetteur.

9. Procédé mis en œuvre par ordinateur d'appairage sans fil selon l'une quelconque des revendications 1 à 8, dans lequel le dispositif récepteur lance un processus d'appairage sans fil uniquement lorsqu'il est dans un état d'appairage.

10. Procédé mis en œuvre par ordinateur d'appairage sans fil selon l'une quelconque des revendications 1 à 9, dans lequel :

     - les circuits de capteur d'impact d'émetteur comprennent un capteur piézoélectrique et les circuits de capteur d'impact de récepteur comprennent un microphone ou un accéléromètre.

11. Procédé mis en œuvre par ordinateur d'appairage sans fil selon l'une quelconque des revendications 1 à 10, dans lequel :

     - le dispositif récepteur est opérationnel entre (i) un état de non-appairage, et (ii) un état d'appairage dans lequel un impact ayant la caractéristique prédéfinie peut être détecté et entraîne la réalisation par le dispositif récepteur du processus d'appairage de dispositif récepteur.

12. Dispositif émetteur (1, 100, 200, 300), comprenant :

     - des circuits électroniques comprenant :

         - un moyen de transmission sans fil pour le transfert sans fil de données à un dispositif externe,
         - des circuits de capteur d'impact destinés à détecter un d'impact exercé sur le dispositif émetteur, et
         - une horloge,

     dans lequel, lorsqu'un impact sur le dispositif émetteur ayant une caractéristique prédéfinie est détecté par les circuits de capteur d'impact, le dispositif émetteur est adapté :

     - à la détermination d'au moins une valeur liée au temps sur la base de l'événement d'impact détecté, et
     - au lancement d'un processus d'appairage sans fil comprenant les étapes de :

         • récupération ou détermination d'une clé K pour le codage et le décodage de données,
         • sur la base de la clé K et d'au moins une valeur liée au temps, calcul d'un indice H,
         • transmission d'un paquet de données comprenant l'indice H et comportant au moins une valeur liée au temps.

13. Dispositif émetteur selon la revendication 14, le dispositif émetteur se présentant sous la forme d'un dispositif d'administration de médicament (100, 200) ou d'un ensemble d'administration de médicament (300, 400), comprenant en outre :

     - un réservoir de médicament ou un moyen destiné à recevoir un réservoir de médicament, et
     - un moyen d'expulsion de médicament comprenant un moyen de réglage de dose permettant à un utilisateur de régler une quantité de dose de médicament à expulser,

les circuits électroniques comprenant en outre :

     - un moyen de capteur adapté à la capture d'une valeur de propriété liée à la quantité de dose de médicament expulsée du réservoir par le moyen d'expulsion pendant un événement d'expulsion, et
     - un moyen de stockage adapté au stockage de données sous la forme d'une pluralité de valeurs de propriété pour créer ainsi un journal.

14. Dispositif récepteur (2), comprenant :

     - des circuits électroniques comprenant :

         - un moyen de réception de données sans fil,
         - des circuits de capteur d'impact destinés à détecter un impact exercé sur le dispositif récepteur,
         - une horloge, et
         - un algorithme stocké pour calculer une clé K sur la base d'un indice H reçu et d'une valeur temporelle liée à l'impact,

dans lequel, lorsqu'un impact sur le dispositif récepteur ayant une caractéristique prédéfinie est détecté par les circuits de capteur d'impact, le dispositif récepteur est adapté :

     - à la détermination d'au moins une valeur liée au temps sur la base de l'événement d'impact détecté, et

- au lancement d'un processus d'appairage sans fil comprenant les étapes de :

  • réception d'un paquet de données comprenant un indice H,
  • sur la base d'un indice H reçu et d'au moins une valeur liée au temps déterminée, calcul et stockage de la clé K.

15. Dispositif récepteur selon la revendication 14, le dispositif récepteur se présentant sous la forme d'un dispositif de stockage comprenant en outre :

  - un moyen de stockage adapté au stockage de données sous la forme d'une pluralité de valeurs de propriété liées à des quantités de doses de médicament pour créer ainsi un journal de doses.

# Fig. 1

PEN — 1

PHONE — 2

$t_{tap}$ ◄------- User taps phone with pen -------► $t_{tap-phone}$

Tap detected by piezo

Tap detected by mic

Calculate hint:
h = key - $t_{tap}$

Random delay ($d_{rnd}$)

Measured delay (d)

Measured delay (d)

$t_{pen}$  Radio packet containing:
tpen, h, data encrypted with key

$t_{phone}$

Estimate delay:
$d = t_{phone} - t_{tap-phone}$

Calculate hint:
h = key - $t_{tap}$

$t_{int}$

Calculate key:
$t_{tap} = t_{pen} - d$
key = $t_{tap}$ + h

$t_{pen1}$  Radio packet containing:
$t_{pen1}$, h, data encrypted with key

Measured delay ($d_1$)

New chance to estimate delay and calculate key as above key using $t_{pen1}$, $d_1$ and h

# Fig. 2

**PEN** — 1

**PHONE** — 2

$t_{tap1}$ — User taps phone with pen — $t_{tap\text{-}phone1}$

Tap detected by piezo

Tap detected by mic

Measured delay $d_0$

$d_{taps}$

$t_{tap2}$ — User taps phone with pen — $t_{tap\text{-}phone2}$

Tap detected by piezo

Tap detected by mic

Estimate delay:
$$d_{taps} = t_{tap2} - t_{tap1}$$

Calculate hint:
$$h = key - f(d_{taps})$$

Radio packet containing:
h, data encrypted with key

Estimate delay:
$$d_0 = t_{tap\text{-}phone2} - t_{tap\text{-}phone1}$$

Calculate key:
$$key = f(d_0) + h$$

EP 3 776 575 B1

**Fig. 3**

| PEN — 1 | PHONE — 2 |
|---|---|

$t_{tap1}$ ◄········· User taps phone with pen ········► $t_{tap\text{-}phone1}$
Tap detected by piezo — Tap detected by mic

Measured delay $d_0$

$t_{tap2}$ ◄········· User taps phone with pen ········► $t_{tap\text{-}phone2}$
Tap detected by piezo — Tap detected by mic

Calculate hint:
$h = key - f(d_{taps}, d_{rnd})$

$d_{taps}$ — Measured delay $d_0$ — Random delay ($d_{rnd}$) — Measured delay ($d$)

$t_{pen}$ — Radio packet containing:
$t_{pen}$, h, data encrypted with key — $t_{phone}$

Estimate delays:
$d_0 = t_{tap\text{-}phone2} - t_{tap\text{-}phone2}$
$d = t_{phone} - t_{tap\text{-}phone2}$

Calculate key:
$t_{tap} = t_{pen} - d$
$key = f(d_0, d) + h$

$t_{int}$ — Measured delay ($d_1$)

$t_{pen1}$ — Radio packet containing:
$t_{pen1}$, h, data encrypted with key

New chance to estimate
delay and calculate key as
above using $t_{pen1}$, $d_0$, $d_1$ and h

EP 3 776 575 B1

## Fig. 4A

## Fig. 4B

**Fig. 5**

**Fig. 6**

**EP 3 776 575 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090318865 A
- WO 2010052275 A
- WO 2016110592 A
- WO 2015071354 A
- WO 2013120776 A
- WO 2014161952 A
- US 20170201931 A
- US 2015117645 A1
- EP 17205309 A
- WO 2018078178 A
- WO 2014128155 A
- EP 2018075639 W